(19) Europäisches Patentamt
European Patent Office
Office européen des brevets

(11) Veröffentlichungsnummer: **0 055 396**
**B1**

(12) **EUROPÄISCHE PATENTSCHRIFT**

(45) Veröffentlichungstag der Patentschrift: **23.01.85**

(21) Anmeldenummer: **81109947.2**

(22) Anmeldetag: **27.11.81**

(51) Int. Cl.⁴: **A 61 K 9/70**, A 61 K 9/12, A 61 K 31/415

(54) **Antimykotische Mittel mit hoher Wirkstoff-Freisetzung in Form von elastischen Flüssig-Pflastern.**

(30) Priorität: **05.12.80 DE 3045915**

(43) Veröffentlichungstag der Anmeldung: **07.07.82 Patentblatt 82/27**

(45) Bekanntmachung des Hinweises auf die Patenterteilung: **23.01.85 Patentblatt 85/4**

(84) Benannte Vertragsstaaten: **AT BE CH DE FR GB IT LI NL SE**

(56) Entgegenhaltungen:
**DE - A - 2 461 406**
**FR - A - 2 275 194**
**US - A - 3 476 853**

**CHEMICAL ABSTRACTS, Band 90, Nr. 16, 16. April 1979 Zusammenfassung 127561x, Seite 378 COLUMBUS, OHIO (US)**

(73) Patentinhaber: **BAYER AG, Konzernverwaltung RP Patentabteilung, D-5090 Leverkusen 1 Bayerwerk (DE)**

(72) Erfinder: **von Bittera, Miklos, Max-Scheler-Strasse 7, D-5090 Leverkusen 3 (DE)**
Erfinder: **Büchel, Karl Heinz, Prof. Dr., Dabringhausener Strasse 42, D-5093 Burscheid (DE)**
Erfinder: **Plempel, Manfred, Dr., Pahlkestrasse 5, D-5600 Wuppertal 1 (DE)**
Erfinder: **Regel, Erik, Ing. grad., Bergerheide 72a, D-5600 Wuppertal 1 (DE)**

**EP 0 055 396 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft neuartige Formulierungen der bekannten antimykotischen Azolderivate, die eine Depot-Wirkung trotz Filmbildung und eine höhere Bioverfügbarkeit der Wirkstoffe aufweisen und dadurch eine Kurzzeittherapie ermöglichen.

Für die Behandlung von Mykosen beim Menschen, vor allem die Mykosen der Haut, sind bereits Zubereitungen von antimykotischen Derivaten bekanntgeworden. Mit diesen Zubereitungen wurden für eine vollständige Sanierung >21 Tage Therapiezeit benötigt.

Um zu einer Verkürzung der Therapiedauer zu kommen, benötigt man, besonders zur Eliminierung der Keime, bzw. um eine mykologische Sanierung zu erzielen, eine gewisse Depot-Wirkung und eine höhere Bioverfügbarkeit der Wirkstoffe. Dafür sind die bekannten Formulierungen nur begrenzt geeignet, weil sich von dem vorhandenen Wirkstoffangebot nur ein kleiner Anteil im Flüssigvolumen am Ort der Infektion löst. Wenn man nun ohne weitere Erhöhung der Wirkstoffkonzentration eine Verkürzung der Therapiedauer, z. B. auf einen Tag bei einmaliger Applikation, erreichen will, muß man für eine optimale Bioverfügbarkeit des Wirkstoffes Sorge tragen.

Es wurde nun gefunden, daß solche Formulierungen antimykotischer Wirkstoffe, die Lösungsmittel, 2—10 Gew.-% Spreitmittel, 1—8 Gew.-% Lösungsvermittler und als Filmbildner einen Celluloseether, insbesondere Hydroxypropylcellulose, die sowohl in Wasser als auch in organischen Lösungsmitteln löslich ist, und außerdem die üblichen Formulierungshilfsstoffe enthalten, eine optimale Freisetzung des Wirkstoffes und damit eine auf einen Tag verkürzte Therapiedauer durch das Erreichen von hohen Konzentrationen des Wirkstoffes ermöglichen. Dieser Effekt wird dadurch erreicht, daß die Wirkung der in den Formulierungen enthaltenen Wirkstoffe durch Spreitöle und Lösungsvermittler und adhärierende Filmbildner-Zugabe erhöht werden und dadurch die Wirkstoff-Freisetzung bis ins Zehnfache gesteigert werden kann. Die erfindungsgemäßen elastischen Flüssig-Pflaster-Formulierungen stellen ein neues Applikationsprinzip zur dermalen Behandlung von Mykosen dar, das neben einer sehr guten Wirksamkeit durch den Verschluß der Infektionsstelle einen Infektionsschutz für die Umgebung darstellt. Besonders gut geeignet sind die erfindunsgemäßen Formulierungen für die Behandlung von Nagelmykosen.

Die erfindungsgemäßen Formulierungen können sowohl Lösungen als auch Sprays sein.

Aus Chem. Abstr. 90 (1979), 127 561x, sind bereits antimykotische Zubereitungen bekannt, die einen Filmbildner, einen Weichmacher und ein Lösungsmittel, jedoch kein Spreitmittel und keinen Lösungsvermittler enthält. Diese Zubereitungen sind jedoch nur schwach antimykotisch wirksam.

Wirkstoffe, die in der erfindungsgemäßen Weise formuliert werden können, sind alle antimykotisch wirksamen Azolderivate, insbesondere Imidazol- und Triazolderivate. Sie sind in den erfindungsgemäßen Mitteln in Mengen von 0,05—1%, vorzugsweise 0,1—1%, vorhanden.

Beispielsweise seien die Verbindungen der nachstehenden Formeln genannt:

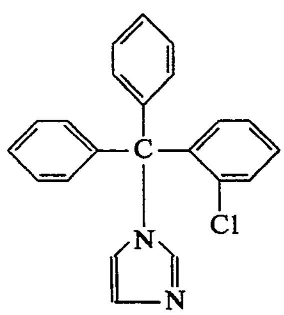

Clotrimazol (I)

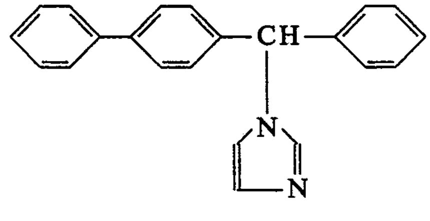

Trifonazol (II)
(auch als Bifonazol bekannt)

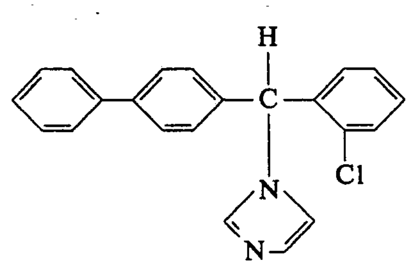

Lombazol (III)

Zahlreiche weitere antimykotisch wirksame Azolderivate sind bekannt aus der DE-OS 2 430 039. Sie können ebenfalls in den erfindungsgemäßen Mitteln als Wirkstoffe dienen. Die geeigneten Spreitmittel gemäß der Erfindung sind folgende:

Isopropylmyristat, Isopropylpalmitat, Isopropylstearat, Capryl/Caprinsäureester von gesättigten Fettalkoholen der Kettenlänge $C_{12}-C_{18}$, wachsartige Fettsäureester wie künstliches Entenbürzeldrüsenfett, Silikonöle,
Isopropylmyristat-Isopropylpalmitat-Isopropylstearat-Gemisch und
Kokosfettsäureisopropylester.

Diese Spreitmittel sind in den erfindungsgemäßen Zubereitungen zu 2 bis 10 Gew.-% enthalten.
Als Lösungsvermittler eignen sich für die erfindungsgemäßen Mittel:

Benzylalkohol, 2-Octyl-dodecanol, Polyethylenglykole, Phthalate, Adipate, Propylenglykol, Glycerin, Di- oder Tripropylenglykol und Wachse.

Diese Lösungsvermittler sind in den erfindungsgemäßen Zubereitungen zu 1 bis 8 Gew.-% enthalten.

Als Filmbildner kommen Celluloseether in Frage, die sich sowohl in Wasser als auch in organischen Lösungsmitteln lösen bzw. anquellen können und nach dem Trocknen eine Art Film bilden.

Besonders geeignet ist Hydroxypropylcellulose.

Weitere geeignete Celluloseether sind z. B. Methylcellulose, Ethylcellulose sowie lösliche Stärken.

Diese Filmbildner werden in Mengen von 10 bis 12 Gewichtsteilen auf 100 Volumenteile Endprodukt eingesetzt.

Als Lösungsmittel sind Wasser und auch alle mit Wasser mischbaren Lösungsmittel geeignet. In Betracht kommen z. B. Alkanole, wie Ethanol und Isopropylalkohol, Propylenglykol, Methylcellosolve, Cellosolve, Ester, Morpholine, Dioxan, Dimethylsulfoxid, Dimethylformamid, Tetrahydrofuran, Cyclohexanon etc.

Es können bei der Herstellung der erfindungsgemäßen Formulierungen ein oder mehrere Lösungsmittel eingesetzt werden.

Bei den Versuchen zur Ermittlung einer optimalen Formulierung können u. a. folgende Hilfsstoffe eingesetzt werden:

Glycerin, Paraffin dickflüssig, Paraffin dünnflüssig, Triethanolamin, Collagen, Allantoin, Novantisolsäure, Parfümöle.

Als weitere Hilfsmittel sind geeignet:

a) Substanzen, die z. B. eine Suspension stabilisieren können, z. B. kolloidale Kieselsäure, Montmorillonite u. a.
b) Tenside (beinhaltet Emulgatoren und Netzmittel), z. B.
   1. anionaktive, wie Na-Laurylsulfat, Fettalkoholethersulfate, Mono/Dialkylpolyglykoletherorthophosphorsäureester-Monoethanolaminsalz;
   2. kationaktive, wie Cetyltrimethylammoniumchlorid;
   3. ampholytische, wie Di-Na-N-lauryl-$\beta$-ininodipropionat oder Lecithin;
   4. nicht ionogene, z. B. polyoxyethyliertes Rizinusöl, polyoxyethyliertes Sorbitan-Monooleat, Sorbitan-Monostearat, Cetylalkohol, Glycerinmonostearat, Polyoxyethylenstearat, Alkylphenolpolyglykolether.
c) Stabilisatoren zur Verhinderung des bei einigen Wirkstoffen eintretenden chemischen Abbaues, wie Antioxydantien, z. B. Tocopherole, Butylhydroxyanisol.
d) Sauer eingestellte wäßrige Lösungen können durch den Zusatz in der Kosmetik üblicher Konservierungsmittel, z. B. p-Hydroxybenzoesäureester, stabilisiert werden.

Wirksamkeits-Testung der erfindungsgemäßen Mittel am Trichophyton-infizierten Meerschweinchen.

Als Testmodell zur vergleichenden Wirksamkeitsprüfung der erfindungsgemäßen Mittel verwendeten wir Trichophyton-infizierte Pirbright-withe-Meerschweinchen mit einem durchschnittlichen Gewicht von 600 g. Die Tiere wurden auf dem Rücken mit einer elektrischen Haarschneidemaschine so geschoren, daß ca. $^1/_{10}$ mm lange Haarstümpfe stehenblieben.

Die Infektion mit Trichophyton mentagrophytes erfolgte durch leichtes Verreiben einer 24 Stunden in Sabouraud-Nährlösung angekeimten Sporensuspension des Erregers auf einer ca. 2 x 2 cm großen Fläche des geschorenen Rückens der Tiere. Aufgetragen wurden pro Tier 0,5 ml Keimsuspension, die $1-3 \times 10^5$ infektiöse Pilzpartikel enthielten.

Bei diesem Infektionsmodus zeigen sich 2—3 Tage post infectionem die ersten Symptome der Dermatophytose als Rötung und Schuppung der Haut. Bei unbehandelten Tieren ist ca. 14 Tage p. i. die

Dermatophytose maximal ausgeprägt. Flächiger Haarausfall und blutige Integument-Defekte innerhalb einer entzündlich veränderten, schuppigen Randzone.

Die zu prüfenden Formulierungen wurden 1mal, am 2. Tag post infektionem, lokal auf die gerötete Infektionsstelle der Tiere appliziert. Es wurden jeweils 0,5 ml der Formulierungen = 5 mg Wirkstoff (1%ige Formulierung) aufgetragen. Die Bewertung des Infektionsablaufs erfolgte täglich bis zum 20. Tag p. i.

Die Ergebnisse sind bei den Beispielen angegeben (+ = schwache Wirkung, ++ = Wirkung, +++ = gute Wirkung, ++++ = sehr gute Wirkung).

In den nachstehenden Beispielen sind Rezepturen für erfindungsgemäße Mittel angegeben. Die einzelnen Komponenten werden bei Zimmertemperatur miteinander vermischt und gehen dabei in Lösung.

M.G. bedeutet Molekulargewicht.

Verwendet man anstelle der erfindungsgemäßen Formulierungen solche, die anstelle von Celluloseethern wasserunlösliche Polymere, z. B. Methacrylate, enthalten, so wird die Mykose verschlimmert.

Verwendet man solche Formulierungen, die neben dem Wirkstoff nur wasserlösliche Celluloseether, aber weder Spreitmittel noch Lösungsvermittler enthalten, erzielt man nur eine schwache Wirkung.

## Beispiel 1

| | |
|---|---|
| Trifonazol | 0,1 g |
| Benzylalkohol | 5,0 g |
| Isopropylmyristat (Spreitmittel) | 6,0 g |
| Hydroxypropylcellulose (M.G. 60 000) | 10,0 g |
| Isopropanol | ad 100 ml |

Wirkung im Meerschweinchen-Test +++ = gute Wirkung.

## Beispiel 2

| | |
|---|---|
| Trifonazol | 1,0 g |
| Benzylalkohol | 4,0 g |
| Isopropylstearat (Spreitmittel) | 10,0 g |
| Hydroxypropylcellulose (M.G. 60 000) | 12,0 g |
| Isopropanol | ad 100 ml |

Wirkung im Meerschweinchen-Test +++ = gute Wirkung.

## Beispiel 3

| | |
|---|---|
| Lombazol | 1,0 g |
| 1,2-Propylenglykol | 1,0 g |
| Isopropylmyristat (Spreitmittel) | 6,0 g |
| Hydroxypropylcellulose (M.G. 60 000) | 10,0 g |
| Isopropanol | ad 100 ml |

Wirkung im Meerschweinchen-Test +++ = gute Wirkung.

## Beispiel 4

| | |
|---|---|
| Lombazol | 0,1 g |
| Benzylalkohol | 5,0 g |
| Isopropylmyristat (Spreitmittel) | 6,0 g |
| Hydroxypropylcellulose (M.G. 60 000) | 10,0 g |
| Isopropanol | ad 100 ml |

Wirkung im Meerschweinchen-Test +++ = gute Wirkung.

Beispiel 5

| | |
|---|---|
| Clotrimazol | 1,0 g |
| Benzylalkohol | 5,0 g |
| Isopropylmyristat (Spreitmittel) | 6,0 g |
| Hydroxypropylcellulose (M.G. 60 000) | 10,0 g |
| Isopropanol | ad 100 ml |

Wirkung im Meerschweinchen-Test + + + + = sehr gute Wirkung.

Beispiel 6

| | |
|---|---|
| Lombazol | 1,0 g |
| Benzylalkohol | 8,0 g |
| Isopropylmyristat/Isopropylstearat/ Isopropylpalmitat (Spreitmittel) | 1,0 g |
| Hydroxypropylcellulose (M.G. 60 000) | 10,0 g |
| Isopropanol | ad 100 ml |

Wirkung im Meerschweinchen-Test + + + = gute Wirkung.

Beispiel 7

| | |
|---|---|
| Clotrimazol | 1,0 g |
| Benzylalkohol | 5,0 g |
| Isopropylmyristat (Spreitmittel) | 6,0 g |
| Methylcellulose | 10,0 g |
| Isopropanol | ad 100 ml |

Wirkung im Meerschweinchen-Test + + + = gute Wirkung.

Sprays

Die nach den Beispielen 1—7 hergestellten Wirkstofflösungen können auch zu Sprays verarbeitet werden. Zu diesem Zweck vermischt man z. B. 60—90% Wirkstofflösung mit 20—40% der gebräuchlichen Treibmittel, z. B. $N_2$, $N_2O$, $CO_2$, Propan, Butan oder Halogenkohlenwasserstoff.

**Patentansprüche für die Vertragsstaaten: BE, CH, DE, FR, GB, IT, LI, NL, SE**

1. Antimykotische Mittel mit höherer Freisetzung der Wirkstoffe, enthaltend Azolderivate, Lösungsmittel und übliche Formulierungshilfsstoffe, dadurch gekennzeichnet, daß sie als Spreitmittel 2 bis 10 Gew.-% Isopropylmyristat, Isopropylpalmitat, Isopropylstearat, Capryl/Caprinsäureester von gesättigten Fettalkoholen der Kettenlänge $C_{12}$—$C_{18}$, wachsartige Fettsäureester, Silikonöle, Isopropylmyristat-Isopropylpalmitat-Isopropylstearat-Gemisch oder Kokosfettsäureisopropylester, als Lösungsvermittler 1 bis 8 Gew.-% Benzylalkohol, 2-Octyl-dodecanol, Polyethylenglykole, Phthalate, Adipate, Propylenglykol-Glycerin, Di- oder Tripropylenglykol oder Wachse und als Filmbildner 10—12 Gewichtsteile Celluloseether auf 100 Volumteile des Endprodukts enthalten.

2. Antimykotische Mittel nach Anspruch 1, dadurch gekennzeichnet, daß sie als Wirkstoff Clotrimazol der Formel

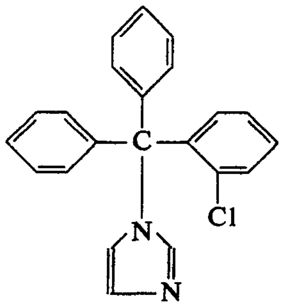

enthalten.

3. Antimykotische Mittel nach Anspruch 1, dadurch gekennzeichnet, daß sie als Wirkstoff Trifonazol der Formel

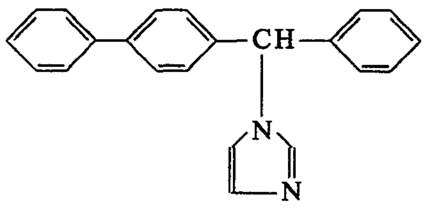

enthalten.

4. Antimykotische Mittel nach Anspruch 1, dadurch gekennzeichnet, daß sie als Wirkstoff Lombazol der Formel

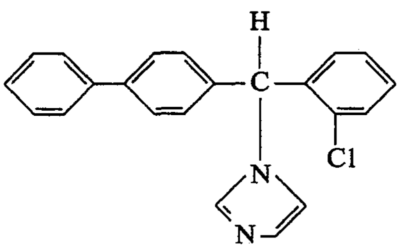

enthalten.

5. Antimykotische Mittel nach Anspruch 1, dadurch gekennzeichnet, daß sie die antimykotischen Azolderivate in Mengen von 0,05 bis 1 Gew.-%, vorzugsweise von 0,1 bis 1 Gew.-%, enthalten.

6. Antimykotisches Mittel nach Anspruch 1, dadurch gekennzeichnet, daß es als Filmbildner Hydroxypropylcellulose enthält.

7. Antimykotisches Mittel nach Anspruch 1, dadurch gekennzeichnet, daß es eine Lösung ist.

8. Antimykotisches Mittel nach Anspruch 1, dadurch gekennzeichnet, daß es ein Spray ist.

**Patentansprüche für den Vertragsstaat: AT**

1. Verfahren zur Herstellung von Antimykotika auf Basis von Azolderivatwirkstoffen und üblichen Formulierungshilfsstoffen, dadurch gekennzeichnet, daß die Wirkstoffe und Formulierungshilfsstoffe zusammen mit 2 bis 10% Masse Isopropylmyristat, Isopropylpalmitat, Isopropylstearat, Capryl/Caprinsäureester von gesättigten Fettalkoholen der Kettenlänge $C_{12}-C_{18}$, wachsartige Fettsäureester, Silikonöle, Isopropylmyristat-Isopropylpalmitat-Isopropylstearat-Gemisch oder Kokosfettsäureisopropylester als Spreitmittel, mit 1 bis 8% Masse Benzylalkohol, 2-Octyl-dodecanol, Polyäthylenglykole, Phtalate, Adipate, Propylenglykol, Glycerin, Di- oder Tripropylenglykol als Lösungsvermittler und mit 10 bis 15% Masse Zelluloseäther als Filmbildner, jeweils bezogen auf die Gesamtmenge, formuliert werden.

2. Verfahren nach Anspruch 1, dadurch gekennzeichnet, daß Hydroxypropylzellulose als Filmbildner eingesetzt werden.

3. Verfahren nach Anspruch 1 oder 2, dadurch gekennzeichnet, daß die Wirkstoffe, insbesondere Clotrimazol, Trifonazol oder Lombazol, in Mengen von 0,05 bis 1% Masse, vorzugsweise von 0,1 bis 1% Masse, bezogen auf die Gesamtmasse, eingesetzt werden.

4. Verfahren nach einem der Ansprüche 1 bis 3, dadurch gekennzeichnet, daß ein Antimykotikum in Form einer Lösung formuliert wird.

5. Verfahren nach Anspruch 4, dadurch gekennzeichnet, daß eine Lösung zur Anwendung als Spray formuliert wird.

**Claims for the Contracting States: BE, CH, DE, FR, GB, IT, LI, NL, SE**

1. Antimycotic agents having a higher degree of release of the active compounds and containing azole derivatives, solvents and customary formulation auxiliaries, characterised in that they contain as spreading agents 2 to 10% by weight of isopropyl myristate, isopropyl palmitate, isopropyl stearate, carprylic/capric acid esters of saturated fatty alcohols of $C_{12}-C_{18}$ chain lenght, waxy fatty acid esters, silicone oils, an isopropyl myristate/isopropyl palmitate/isopropyl stearate mixture or coconut oil acid isopropyl ester, as solublisers 1 to 8% by weight of benzyl alcohol, 2-octyl-dodecanol, polyethylene glycols, phthalates, adipates, propylene glycol, glycerol, di- or tripropylene glycol or waxes and as film-forming agents 10—12 parts by weight of cellulose ether per 100 parts by volume of the end product.

2. Antimycotic agents according to Claim 1, characterised in that they contain clotrimazole of the formula

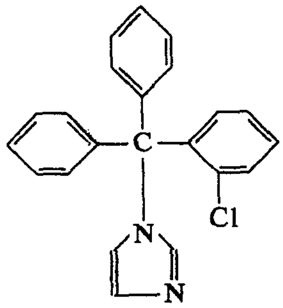

as the active compound.

3. Antimycotic agents according to Claim 1, characterised in that they contain trifonazole of the formula

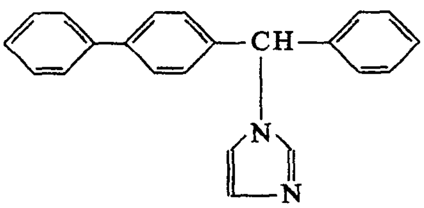

as the active compound.

4. Antimycotic agents according to Claim 1, characterised in that they contain lombazole of the formula

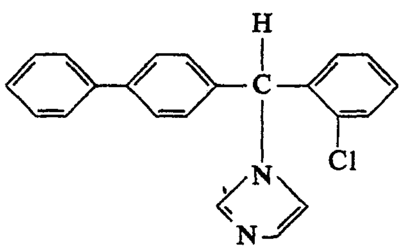

as the active compound.

5. Antimycotic agents according to Claim 1, characterised in that they contain the antimycotic azole derivatives in quantities of 0.05 to 1% by weight, preferably 0.1 to 1% by weight.

6. Antimycotic agent according to Claim 1, characterised in that it contains hydroxypropylcellulose as the film-forming agent.

7. Antimycotic agent according to Claim 1, characterised in that it is a solution.

8. Antimycotic agent according to Claim 1, characterised in that it is a spray.

**Patent Claims for the Contracting State: AT**

1. Process for the preparation of antimycotics based on azole derivative active compounds and customary formulation auxiliaries, characterised in that the active compounds and formulation auxiliaries are formulated together with 2 to 10% by weight of isopropyl myristate, isopropyl palmitate, isopropyl stearate, caprylic/capric acid esters of saturated fatty alcohols of $C_{12}-C_{18}$ chain length, waxy fatty acid esters, silicone oils, an isopropyl myristate/isopropyl palmitate/isopropyl stearate mixture or coconut oil acid isopropyl ester as spreading agents, with 1 to 8% by weight of benzyl alcohol, 2-octyl-dodecanol, polyethylene glycols, phthalates, adipates, propylene glycol, glycerol, di- or tripropylene glycol as solubilisers and with 10 to 15% by weight of cellulose ethers as film-forming agents, based in each case on the total weight.

2. Process according to Claim 1, characterised in that hydroxypropylcellulose is used as the film-forming agent.

3. Process according to Claim 1 or 2, characterised in that the active compounds, in particular clotrimazole, trifonazole or lombazole, are used in quantities of 0.05 to 1% by weight, preferably 0.1 to 1% by weight, based on the total weight.

4. Process according to one of Claims 1 to 3, characterised in that an antimycotic is formulated in the form of a solution.

5. Process according to Claim 4, characterised in that a solution is formulated for use as a spray.

**Revendications pour les Etats contractants: BE, CH, DE, FR, GB, IT, LI, NL, SE**

1. Agents antimycotiques à plus haute libération des substances actives, contenant des dérivés d'azoles, des solvants et des substances auxiliaires habituelles de formulation, caractérisés en ce que, comme agents d'étendage, ils contiennent 2 à 10% en poids de myristate d'isopropyle, de palmitate d'isopropyle, de stéarate d'isopropyle, d'ester caprylique/acide caprique, d'alcools gras saturés d'une chaîne en $C_{12}-C_{18}$, d'esters d'acides gras cireux, d'huiles de silicones, du mélange de myristate d'isopropyle/palmitate d'isopropyle/stéarate d'isopropyle ou d'ester isopropylique d'acides gras de coco; comme unisseurs, ils contiennent 1 à 8% en poids d'alcool benzylique, de 2-octyl-dodécanol, de polyéthylène-glycols, de phtalates, d'adipates, de propylène-glycol-glycérine, de di- ou de tripropylène-glycol ou de cires et, comme agents filmogènes, ils contiennent 10—12 parties en poids d'éthers de cellulose pour 100 parties en volume du produit final.

2. Agents antimycotiques suivant la revendications 1, caractérisés en ce que, comme substance active, ils contiennent le clotrimazol de formule:

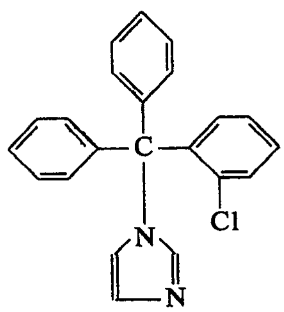

3. Agents antimycotiques suivant la revendication 1, caractérisés en ce que, comme substance active, ils contiennent le trifonazol de formule:

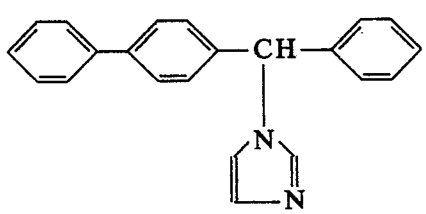

4. Agents antimycotiques suivant la revendications 1, caractérisés en ce que, comme substance active, ils contiennent le lombazol de formule:

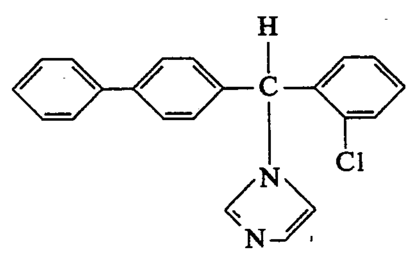

5. Agents antimycotiques suivant la revendication 1, caractérisés en ce qu'ils contiennent les dérivés d'azoles antimycotiques en quantités de 0,05 à 1% en poids, de préférence, de 0,1 à 1% en poids.

6. Agent antimycotiques suivant la revendication 1, caractérisé en ce que, comme agent filmogène, il contient l'hydroxypropyl-cellulose.

7. Agent antimycotique suivant la revendication 1, caractérisé en ce qu'il est une solution.

8. Agent antimycotique suivant la revendication 1, caractérisé en ce qu'il est une pulvérisation.

**Revendications pour l'Etat contractant: AT**

1. Procédé de préparation d'antimycotiques à base de dérivés d'azoles comme substances actives et de substances auxiliaires habituelles de formulation, caractérisé en ce que les substances actives et les substances auxiliaires de formulation sont mises en formulation avec 2 à 10% en poids de myristate d'isopropyle, de palmitate d'isopropyle, de stéarate d'isopropyle, d'ester caprylique/acide caprique, d'alcools gras saturés d'une chaîne en $C_{12}-C_{18}$, d'esters d'acides gras cireux, d'huiles de silicones, du mélange de myristate d'isopropyle/palmitate d'isopropyle/stéarate d'isopropyle ou d'ester isopropylique d'acides gras de coco comme agents d'étendage, 1 à 8% en poids d'alcool benzylique, de 2-octyl-dodécanol, de polyéthylène-glycols, de phtalates, d'adipates, de propylène-glycol, de glycérine, de di- ou de tripropylène-glycol ou de cires comme unisseurs et 10—15% en poids d'éthers de cellulose comme agents filmogènes, chaque fois par rapport au poids de l'ensemble.

2. Procédé suivant la revendication 1, caractérisé en ce que l'hydroxypropyl-cellulose est mise en oeuvre comme agent filmogène.

3. Procédé suivant la revendication 1 ou 2, caractérisé en ce que les substances actives, en particulier le clotrimazol, le lombazol ou le trifonazol sont mises en oeuvre en quantités de 0,05 à 1% en poids, de préférence, de 0,1 à 1% en poids par rapport au poids de l'ensemble.

4. Procédé suivant l'une des revendications 1 à 3, caractérisé en ce qu'un antimycotiques est présenté sous la forme d'une solution.

5. Procédé suivant la revendication 4, caractérisé en ce qu'une solution est présentée sous la forme d'une pulvérisation en vue de son application.